(19) Europäisches Patentamt
European Patent Office
Office européen des brevets

(11) Publication number: **0 261 693**
**A1**

(12) **EUROPEAN PATENT APPLICATION**

(21) Application number: 87114072.9

(22) Date of filing: 25.09.87

(51) Int. Cl.⁴: **A61K 9/52**, A61K 31/785, A61K 9/68

(30) Priority: 26.09.86 US 912902

(43) Date of publication of application: 30.03.88 Bulletin 88/13

(84) Designated Contracting States: AT BE CH DE ES FR GB GR IT LI LU NL SE

(71) Applicant: WARNER-LAMBERT COMPANY
201 Tabor Road
Morris Plains New Jersey 07950(US)

(72) Inventor: Ghebre-Sellassie, Isaac
359 Dell Place
Stanhope New Jersey 07874(US)
Inventor: Gordon, Robert H.
134 Lincoln Avenue
Dover New Jersey 07801(US)
Inventor: Fawzi, Mahdi B.
11 Timberline Drive
Flanders New Jersey 07836(US)

(74) Representative: Mansmann, Ivo
c/o Gödecke AG - Patentabteilung Postfach
569 - Mooswaldallee 1-9
D-7800 Freiburg(DE)

(54) **Treated lipid regulator.**

(57) Cholestyramine can be administered orally either alone or in combination with one or more other beneficial agent(s) in compositions in which the cholestyramine has been suitably pretreated.

EP 0 261 693 A1

## TREATED LIPID REGULATOR

Cholestyramine is a well-known ion-exchange resin which has pharmaceutical utility as an antihyperlipemic agent which lowers serum cholesterol. During normal digestion, most of the bile acids secreted into the duodenum are recaptured at specific ileal receptor sites and returned to the liver via the enterohepatic circulation. Cholestyramine resin combines with the bile acids to form an insoluble complex that is secreted in the feces, thereby leading, eventually, to a decrease in serum cholesterol levels.

While cholestyramine is highly effective, it is gritty or sandy in texture, making it unpleasant in appearance and mouthfeel. One result of this unpleasantness is reduced patient compliance in that individuals who ingest the resin on a regular basis may avoid taking all of the resin they need.

Combination products, ie., compositions which contain cholestyramine and one or more other beneficial substances or drugs are difficult to formulate because of the ionic character of the resin. For instance, an acidic drug cannot be readily administered along with cholestyramine because the drug can bind to the ionic sites on the resin, and thereby reduce the effectiveness of both agents.

It has been discovered that useful compositions containing cholestyramine or other lipid modifier(s) of ionic character can be produced by pretreating the ionic lipid modifier using a certain regimen. Optionally, the treated lipid modifier(s) can be combined with at least one other beneficial substance, e.g. a second lipid lowering agent.

In one embodiment, cholestyramine resin is granulated to yield granules whose particle size ranges from about 10 to about 100 microns. These granules are then coated with a material which is acid resistant, but soluble in the intestinal juice of the duodenum or other portion of the intestines where bile acids are secreted. This pretreated cholestyramine can then be administered alone or in combination with other drugs or beneficial substances.

The compositions of the invention have several advantages over known compositions containing cholestyramine resin. The pretreated cholestyramine resin is in a form which makes it readily includable into various dosage forms such as candy bars, chewing gum, liquid and semi-solid suspensions, sprinkle systems, tablets capsules and the like.

Furthermore, the coating process used in the pretreatment virtually assures that the cholestyramine or other ionic component will not react to any significant extent before it reaches the proper location in the gastrointestinal tract so that its efficacy is maximized.

These and other aspects and advantages of the invention will be readily apparent upon consideration of the following description of the invention.

The invention involves a composition and a method by which the interaction between cholestyramine and other drugs, especially other lipid regulating drugs, if any, is minimized when the cholestyramine is ingested alone or in combination by human subjects.

The compositions of the invention contain:

a) at least one lipid regulator which has been pretreated to render it stable until it reaches the proximal section or other appropriate areas of the intestines, and other, optionally,

b) at least one other therapeutically active substance.

The invention is also concerned with methods of use of pretreated lipid regulator(s) alone or in combination with other drug(s) for the preparation of pharmaceutical compositions for hyperlipidemic therapy. The composition or series of compositions are formulated such that the active or reactive agents in each drug is absorbed with maximum efficiency in the body.

It is preferred that the cholestyramine or other lipid regulator of ionic character be pretreated via a process which involves:

(1) granulation; and

(2) coating with an acid resistant coating material.

It is highly preferred that the coating material used in step (2) be soluble in the juices of the proximal a duodenal region of the intestine, i.e., in that portion of the intestine in which bile acids are present in significant amounts. By "significant amounts" applicants mean quantities in which bile acids can be effectively bound to the reactive sites of an ionic lipid modifier such as cholestyramine so that their concentration in the bloodstream is lowered.

The drug combinations of the invention will combine a lipid regulator, eg., cholestyramine and at least one other beneficial or therapeutic material.

The lipid regulator(s) useful in the invention can be selected from any of those conventionally employed whose ionic character render them suitable for binding bile salts and/or other fatty materials. Useful lipid regulators include those which act by ionic mechanisms to remove one or more fatty substances from the bloodstream and to increase the concentration of same in fecal excretions.

Preferred lipid modifiers of the ionic type are cholestyramine, colestipol, and the like and their pharmaceutically acceptable salts, e.g, cholestipol HCl. Combinations of cholestyramine and other anionic exchange resins, are contemplated. Combinations containing lipid modifiers which do not act by ionic mechanisms are also comtemplated, e.g, gemfibrozil and cholestyramine can be combined.

Cholestyramine is a pharmacologically important anionic-exchange resin. The basic quaternary ammonium-exchange functionalities in the resin are attached to a styrene-divinylbenzene copolymer skeleton.

Its structure is:

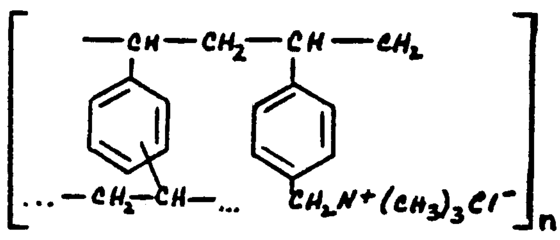

wherein n is a function of molecular weight.

It has been demonstrated in animals and clinically in man that cholestyramine is capable of increasing the fecal excretion of endogenous bile salts, thereby significantly decreasing the extent of absorption of fats and fatty materials. This resin also possesses the ability to lower plasma cholesterol levels by binding bile-salt anions in the small intestine. One article which discusses the resins binding capacity is W. J. Johns and T. R. Bates, "Quantification of the Binding Tendencies of Cholestyramine I; Effect of Structure and Added Electrolytes on the Binding of Unconjugated and Conjugated Bile-Salt Anions,"Journal of Pharm. Science, vol. 58, No. 2, February, 1969, pp. 179-83. The content of that article is hereby incorporated by reference.

Applications contemplate formulations in which cholestyramine and/or other lipid modifiers of ionic character are used in combination with one or more agents which inhibit the production and/or absorption of cholesterol. Suitable agents include, but are not limited to, fabric acid derivatives, cholereductase inhibitors, antihyperlipoproteinemics and the like.

Useful lipid lowering/regulating agents include--but are not limited to--gemfibrozil, dextrothyroxine, the fibrates, e.g, fenofibrate, clofibrate, bezafibrate, ciprofibrate, mevinolin, synvinolin, niacin, hormonal preparations, e.g, T3-and/or T4-containing agents, fish oils and/or extracts of such oils, eptastin, the neomycins, probucol, nicotinic acid, and the like, as well as their pharmaceutically acceptable salts and esters, e.g, dextrothyroxine sodium, gemfibrozil HCl and the like. Gemfibrozil and its pharmaceutically acceptable derivatives are preferred.

Gemfibrozil and its analogs are discussed in U.S. patents 3,674,836 and 4,126,637, the disclosures of which are hereby incorporated by reference.

Mixtures of lipid moderators which are not of ionic character are operable, as are mixtures which contain a plurality of ionic-type moderators and a plurality of one or more other types.

The quantities in which the lipid reducing agent(s) will be present in the final therapeutic compositions will vary depending upon the nature of the agents(s) selected and the desired therapeutic effect to be achieved.

In general, in compositions containing cholestyramine, the concentration of cholestyramine will be from about 5 to about 80, preferably about 30 to about 60 weight percent, based on total composition weight.

Unless stated otherwise, all percentages stated herein are weight percentages based on total composition weight.

If a second lipid modifier is used --ie., one which is not of ionic character --it will generally be present in a concentration of about 5 to about 80, preferably about 30 to about 60, weight percent by weight.

In addition to cholestyramine and/or other lipid lowering agent(s), the compositions of the invention can also contain a wide variety of other drugs or beneficial substances.

Suitable categories of drugs that may be employed in combination with the instant treated lipid moderator(s) may vary widely and generally represent any stable drug combination. Illustrative categories and specific examples include:

a) Antitussives, such as dextromethorphan, dextromethorphan hydrobromide, noscapine, carbetapentane citrate, and chlophedianol hydrochloride;

b) Antihistamines, such as chlorpheniramine, maleate, phenindamine tartrate, pyrilamine maleate, doxylamine succinate, and phenyltoloxamine citrate

c) Decongestants, such as phenylephrine hydrochloride, phenylpropanolamine hydrochloride, pseudoephedrine, hydrochloride ephedrine;

d) Various alkaloids, such as codeine phosphate, codeine sulfate and morphine; and

e)Mineral and vitamin supplement such as potassium chloride and calcium carbonates.

These materials may be used singly or in combination within the ranges specified above.

The weight percent of the non-lipid modifying drug or salt thereof, based on the weight of the total composition, will be form about 2% to about 80% and preferably about 5% to about 50% by weight, which amounts can vary depending upon the therapeutic dosage permitted.

The drugs, minerals, and vitamins discussed above can be employed in any of their pharmaceutically acceptable forms. Thus, salts, eg., hydrochlorides, borates, pamoates, and the like as well as other analogs, metabolites and/or pro drugs related to the same may be used. Mixtures are operable.

One preferred group of materials to be combined with the treated lipid modifiers of the invention in orally administrable compositions includes phenylbutazone, warfarin, and chlorthiazide, as well as tetracycline, penicillin G, phenobarbital, thyroid and thyroxine preparations, digitalis chlorpropamide $^{25}S$, niacin -6$^{14}C$, ascorbic acid, aspirin, salicylic acid, phenoborbitol $^{14}C$, sulfadiazine, linocomycin HCl, tetacycline HCl and the like and their pharmaceutically acceptable analogs, e.g. salts. Combinations of these drugs can be employed.

The process by which the primary ionic lipid reducer is treated in order to maximize its compatability with other drugs in the system is a novel one.

The granulation step involves agglomeration or other suitable processing to yield granules or other generally spherical particles whose size range from about 30 to about 100, microns. Suitable devices for the granulation step are well known in the art. If the lipid modifier is supplied in a form which is suitable for coating, the granulation step may be omitted, i.e, granulation is optional.

Following granulation, the particles are then coated with a pharmaceutically acceptable material which is acid resistant, so that the granules remain coated as they pass through the stomach after ingestion. Such materials are generally termed enteric coating materials and are well known in the art. Mixtures of such materials can be used.

It is highly preferred that the acid resistant coating also be soluble in that region of the intestines in which bile salts and other fatty substances can be absorbed, or bound, by the resin, so that they are then taken out of the bloodstream and excreted along with fecal matter. Suitable coating materials are commercially available materials, or combinations of some, which have requisite solubility.

In general, the coating material should be insoluble at the pH generally found in the stomach, but soluble at the pH general found at the proximal intestine. In general, the coating material should be insoluble at pH's less than 5, preferably pH's less than 4, but soluble at the pH of the proximal intestine, ie, about 4 to about 7, preferably about 4 to about 5.

Preferred coating materials are polymers or polymer precursors (e.g monomers and/or resin intermediates) whose films have the solubility properties discussed above. Mixtures are operable.

The method by which the coating material(s) is applied to the primary lipid regulator is not crucial. It is sufficient for the purposes of this invention that this lipid regulator be coated, encapsulated or otherwise treated with the coating material so that little or no association of the ionic lipid regulator takes place in an acid environment, i.e., in the stomach.

Optionally, one or more of the other drugs employed in the combinations of the invention can be suitably pretreated. One preferred treatment involves the taste-masking system set out in U.S.SN. 811,601 (W-L PD #3456-7-DAS).

The final dosage forms in which the drug combinations of the invention will be delivered can vary broadly. Since the compositions are to be administered orally, it is contemplated that any system which affords ingestion via the gastro-intestinal tract can be used. Thus formulations for ingestible tablets, pellets, sprinkles, suspensions, gels, candy bars, chewy candy, chewing gums, lozenges, and the like are contemplated. combinations of these, e.g., lozenges with chewing candy centers, are useful.

Two preferred dosage forms are edible candy formulations and chewing gum formulations. Such formulations can contain fillers, waxes, sweeteners, stabilizers, flavoring agents, fragrance enhancers, processing aids, and the like, which are conventionally used in the confectionery arts. For example, suitable sweeteners include aspartame, saccharine, glucose, sucrose, fructose, xylitol, and the like and combinations thereof.

Excipients, such as fillers, stabilizers and other additives conventionally employed in the pharmaceutical industry and in the candy and confectionery industry, can be used in suitable amounts in the compositions of the invention.

The dosage forms produced in accordance with the invention will be employed to deliver drugs to human recipients in suitable quantites for the therapeutic effect(s) desired, i.e., at dosage levels which are consistent with the therapeutic needs of the patient and the desires of his or her physician. Thus, daily dosage levels are well-known for the lipid regulators employed in the invention and need not be spelled out here. Typically, reference sources such as the Physicians' Desk Reference, and drug manufacturer's specifications and instructions can be consulted to determine proper drug concentration levels and/or dosage regimens for the combinations of the invention.

## Examples

The following examples illustrate the invention:

## Example I

This example shows the effect of pH on the binding of gemfibrozil to untreated cholestyramine.

Gemfibrozil solutions were prepared in 0.05 M acetate buffer, pH 4.5, and 0.05 M phosphate buffer, pH 6.0 and 7.5 as follows:

Gemfibrozil solutions were prepared by adding an excess of gemfibrozil to the desired volume of buffer and agitating overnight on a shaker at room temperature. The resulting suspension was vacuum filtered. Gemfibrozil concentration was determined from absorbance at 280 nm, using

$$A^{1\%}_{1\,cm} = 65.6$$

Ultraviolet absorbance measurements were obtained on a Beckman DU-7 spectrophotometer.

Optical rotation measurements at the sodium D line were made with a Rudolph Auto-Pol 2.

All experiments were conducted (unless otherwise noted) in 50 or 15 ml polypropylene centrifuge tubes with plug and caps. The mixtures were always agitated at 75 RPM on a Vanderkamp Sustained Release Apparatus heated to 37 |-C.

Sodium Glycocholate: Sigma; Lot # 75F-5083

cholestyramine: Rohm & Haas, Amberlite - IRP 276; Lot #6X686

The concentration of gemfibrozil in each buffer, determined from absorbance at 280 nm was: pH 4.5, 0.014 mg/ml; pH 6.0, 0.150 mg/ml; pH 7.5, 1.711 mg/ml. Cholestyramine (10 mg) was added to 20 ml of each gemfibrozil solution and the mixtures were agitated for 2 hours. The mixtures were filtered and the concentration of unboud gemfibrozil was determined.

The solubility of gemfibrozil at pH 4.5 is very low, as shown in Table I. Therefore, despite the fact that gemfibrozil has a high affinity for cholestyramine, very little gemfibrozil is available for binding at this pH. At pH 6,0 paralleling the increase in gemfibrozil solubility (Table I) is an increase in the amount bound. At PH 7.5 the amount of gemfifibrozil in solution is greater than the amount needed to saturate the resin and saturation, indeed, occurs. It is of interest that pH's of 4.5 and 6.0, the resin does not bind 100% of the available gemfibrozil, despite the fact that unoccupied binding sites are present.

TABLE I: Binding of Gemfibrozil to Cholestyramine at Various pH's.

| pH | mmol Gemfibrozil Bound/g Resin |
|---|---|
| 4.5 | 0.11 |
| 6.0 | 0.60 |
| 7.5 | 2.83 |

Example II

This example shows the effect of pH on the binding of gemfibrozil which has been treated in accordance with the invention. The experimental procedure was the same as in Example I. The coated material contained fifty percent active resin.

Table II: Binding of Gemfibrozil to Treated Cholestyramine

| pH | mmol Gemfibrozil Bound/g Resin |
|---|---|
| 4.5 | 0.06 |
| 6.0 | 0.66 |
| 7.5 | 2.83 |

Reasonable variations, such as those which would occur to a skilled artisan, can be made herein without departing from the scope of the invention.

**Claims**

1. A pharmaceutical composition which contains at least one lipid regulator which has been pretreated to render it stable until it reaches the proximal section of the intestines.
2. The composition of Claim 1 wherein the lipid regulator is of ionic character.
3. The composition of Claim 2 which also contains a drug which in not a lipid regulator.
4. The composition of Claim 3 wherein the lipid regulator is cholestyramine.
5. A candy formulation containing the compositions of Claims 1 to 4.
6. A chewing gum formulation containing the compositions of Claims 1 to 4.
7. Method of use of a pretreated lipid regulator according to claims 1 to 4 alone or in combination with other drugs for the preparation of pharmaceuticals for treating hyperocholesteremia.

Claims for the following Contracting States : AT, ES, GR

1. Method of use of a lipid regulator which has been pretreated to render it stable until it reaches the proximal section of the intestines alone or in combination with other drugs for the preparation of pharmaceuticals for treating hypercholesteremia,
2. A method according to claim 1 wherein the lipid regulator is of ionic character.
3. A method according to claim 1 wherein the lipid regulator is cholestyramine.

European Patent Office

# EUROPEAN SEARCH REPORT

Application number

EP 87 11 4072

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | EP-A-0 077 956 (TANABE SEIYAKU CO.)<br>* Page 10, lines 9-16; page 11, line 13; page 11, lines 23-27; claims 2,3 *<br>--- | 1-7 | A 61 K 9/52<br>A 61 K 31/785<br>A 61 K 9/68 |
| X,Y | FR-A-2 197 542 (MERCK & CO.)<br>* Page 1, lines 1-8; page 4, line 30 - page 5, line 10 *<br>--- | 1-4,7 | |
| X | CHEMICAL ABSTRACTS, vol. 96, no. 8, 22nd February 1982, pages 370-371, abstract no. 57772q, Columbus, Ohio, US; & JP-A-81 142 212 (RIKEN VITAMIN OIL CO., LTD) 06-11-1981<br>* Whole abstract *<br>--- | 1,7 | **TECHNICAL FIELDS SEARCHED (Int. Cl.4)** |
| X | EP-A-0 166 542 (AMANO PHARMACEUTICAL CO.)<br>* Page 1, line 24 - page 2, line 7; page 237, example 10; page 238, example 11 *<br>----- | 1,7 | A 61 K |

The present search report has been drawn up for all claims

| Place of search | Date of completion of the search | Examiner |
|---|---|---|
| THE HAGUE | 05-11-1987 | BERTE M.J. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03.82